# EUROPEAN PATENT APPLICATION

(11) **EP 4 815 631 A1**
(43) Date of publication of application: **30.09.2026**
(21) Application number: 25165825.8
(22) Date of filing: 25.03.2025
(51) Int. Cl.: H05B 47/105

(54) **CONTROL DEVICE, SYSTEM AND METHOD FOR DRIVING A LIGHT SOURCE TO CALM DOWN A PERSON**

(71) Applicant: ZG Lighting Netherlands B.V., 5038 ED Tilburg (NL)
(72) Inventor: Lombardo, Bertrand, 6851 Dornbirn (AT)
(74) Representative: Rupp, Christian

(57) **Abstract**

The invention concerns a control device (2), a system (1) and a method for driving a light source (3) to calm down a person. The control device (2) receives at least one input signal indicative of a calming demand. The control device analyses the received at least one signal to recognize a calming demand and generates, in case that a calming demand can be recognized by the evaluation of the at least one input signal, a control signal for controlling an amplitude modulation of emitted light. The control signal includes information on a frequency of the amplitude modulation, and the control device (2) outputs the generated control signal. The control device (2) outputs the control signal and based on the control signal, the light source (3) is controlled to emit light of modulated light intensity with decreasing modulation frequency.

## Description

The invention regards a control device, a system and a method for controlling light emission produced by light source with light emission characteristics suitable to calm down a person in order to reduce a stress level of the person.

It is known that environmental conditions can influence the comfort and stress level of persons. For example, people moving in great darkness usually feel more anxious than in a well illuminated area, especially when streets are empty and people are in an unknown area. Attempts have been made to determine anxiety or panic of people using physiological sensors and, based on the determined anxiety or panic level of the person, change illumination of an area in which the person currently moves. An example for such a system can be found in WO 2017/102416 A1. The system described in this document exploits the fact that pedestrians who walk on badly illuminated roads or pavements tend to be anxious or stressed, and such bad feelings can be avoided or at least alleviated by increasing the light level in the environment of the person. Accordingly, streetlights are controlled in response to measured physiological parameters, which allow to determine anxiety of a person.

In addition, it is known that upcoming panic or an increased level of stress can be reduced if the person concerned is able to control its respiratory rate. Intentionally breathing calmly has an effect on the person's well-being, which, in turn, positively influences other physiological parameters like heart rate and blood pressure. Bringing a cyclic signal with a frequency approximately corresponding to the respiratory rate to attention of a stressed person may help to influence the person such that his or her respiratory rate tends to follow the frequency of the cyclic signal.

It is evident that a reduction of the heart rate and blood pressure necessarily leads to an improved health condition. Avoiding critically high heart rates and blood pressure caused by stress is specifically important for people in overall critical condition, for example, because of heart disease or during recovery after a surgery. However, especially in these situations people tend to be susceptible to panic or at least an increased stress level.

In a clinical environment, patients currently have to request for help and clinic personnel has to look for the patients at and calm them down. To pre-empt such situations it is even possible that the patients are given tranquilizers although not undoubtedly necessary.

In addition, there are many situations in which persons themselves are well aware that lowering the stress level would increase their well-being, even outside a clinical environment. This may be the case after a hard day at work when coming home or during a wellness therapy. There are a lot of different situations in which the reduction of the stress level or panic situation is desirable.

It is therefore an object of the present invention to assist a person in reducing its stress level without the necessity of other people interacting with the concerned person.

This object is achieved with a control device controlling light emission, a system for controlling light emission and a respective method for driving the light source to calm down a person. The control device, system and method according to the invention are defined in the attached independent claims. Advantageous embodiments and additional aspects are defined in the dependent claims.

According to the invention, there is provided a control device for controlling light emission of light source assisting in calming down a person. The control device comprises an interface for receiving at least one input signal indicative of a calming demand. Such input signal might be a measured physiological parameter of the person allowing an analysis and determination of a stress level of the person, or a trigger signal in case of controlling light emission in order to calm down the person is directly initiated by the concerned person or another person. This might be advantageous in a situation, when the concerned person deliberately wants to relax or another person recognizes that the concerned person has an increased stress level. In case that a calming demand is recognized, either by receiving such trigger signal as mentioned above or such camming demand is recognized from a respectively received signal indicative of a stress situation of a person, the control device generates a control signal for controlling an amplitude modulation of emitted light. The control signal includes information on a frequency decrease over time of the amplitude modulation. The control signal is then output by the control device in order to enable operating a light source in accordance with the control signal. In essence and based on the control signal, the light source is controlled to operate with modulated light intensity, wherein the modulation frequency is reduced over time.

With the present invention it is thus possible to generate light emission with a modulated intensity, wherein the frequency of the intensity modulation decreases with time and the intensity variation slows down. As indicated above, a person paying attention to such light emission tends to breathe with a respiratory rate following the frequency of the modulated light intensity. Accordingly, when the light intensity variation slows down, the concerned person is inclined to slow down breathing. Controlling the respiratory rate to slow down has a positive effect on the well-being of the concerned person. In addition, this will also cause a reduction of the heart rate and/or blood pressure, which furthermore reduces the risk of, for example, getting a heart attack.

The system for emitting light with a modulated intensity, the frequency of which is lowered over time, comprises the control device described above, at least one signal source configured to transmit a signal indicative of a calming demand to the control device, a light source and the driver for driving the light source in accordance with the control signal. The driver receives the control signal generated by the control device and drives the light source in response to the control signal and in accordance with the information included in the control signal as explained above. The signal source may be a button or switch providing the trigger signal as input signal in case of deliberately initiating the calming assistance by the light source. In addition or alternatively, the signal source may comprise at least one sensor for sensing at least one physiological parameter of the concerned person. In that case, the respective sensor signal is the input signal for the control device. The measured values of the respective physiological parameter(s) allow an evaluation and, in the end determination of a stress level of the concerned person.

Advantageously, the control device is configured to determine such stress level by evaluating the measured values of the physiological parameters based on the at least one input signal received from the one or more sensors. The control device is configured to determine the stress level and, based thereon, identify a calming demand. For example, stress level may be associated with a value between 0 and 100, and a threshold can be set in order to compare the determined stress level. In case that the determined stress level exceeds the threshold, a calming demand may be recognized. The control device can then automatically start generating the control signal as described above. Obviously, the control device can be configured to generate a control signal in response to receiving the trigger signal as the input signal but also in response to determining the calming demand from input signals received from the sensors as explained.

One advantage of the control device being able to determine a calming demand from the input signal is the possibility to use conventional signal sources such as physiological sensors or buttons to initiate the generation of the control signal by the control device. This allows to make use of already existing components to be included into the inventive system. In particular, in controlled environments, for example a hospital, physiological parameters of persons are monitored regularly. Thus, the values of physiological parameters measured by the sensors can be used as input signals for the control device. The control device then can process the received input signals and determine whether the stress level of the person exceeds a certain threshold. The threshold may be adjustable.

According to a basic configuration, the control device comprises a memory storing a preset frequency decrease rate. Starting from an initial modulation frequency for modulating the light emission intensity, the rate at which the frequency of the modulation is reduced follows a fixed schedule. The preset frequency decrease rate may be adjustable in order to accomplish for different needs of the concerned person. Using such a default frequency decrease rate allows to operate the system even in case that no monitoring of physiological parameters of the concerned person is done or the measured values cannot be used.

According to a preferred embodiment, the control device is configured to repetitively determine the frequency decrease of the amplitude modulation based on the at least one input signal. With this functionality, it is possible to take into account the latest measurement values of the physiological parameters and adjust the assistance to the progress. Accordingly, if the person already calms down, this will be taken into consideration by referring to the actual value of the respiratory rate in case that the breathing frequency of the person is measured with a respective sensor. In case that no new values are available, because the assistance was triggered by pushing a button but without inputting values of physiological parameters, the control device will automatically use the default settings for decreasing the frequency of the modulation.

According to another preferred embodiment, the control device is further configured to include color information for the light to be omitted into the output control signal. Controlling the color of the modulated light output in the end by the light source allows to draw the attention of the concerned person towards the light source when coming down this assisted by starting modulation of the intensity of the light emission. It is specifically advantageous if a color scheme is used enhancing the calming effect of slowing down the frequency of the modulation. For example, the light source is switched to emit green light when the calming sequence is started. Switching the color of the emitted light, for example to green, even allows to use an already operating light source for assisting the person to come down. It is evident that such color information may also be used in case that the light source is only put into operation when a calming demand is recognized and the control signal is generated. In that case, the light source is switched on in accordance with the control signal output by the control device. This means that the light source is switched on and operated to output light with modulated intensity in accordance with the control signal and, preferably, with a dedicated color, for example, green.

The color information may change over time and the color change may depend on the stress level achieved. This means that in response to input signals indicative of a certain stress level, the color information included in the control signal is changed to cause a change of color of the light emitted by the light source. For example, starting with emission of green light, the color may successively change to white light when approaching the target stress level. The transition from green light to white light may be continuous or stepwise.

Changing the color of the emitted light when starting the process of assisting a person in calming down is also advantageous in that it helps to draw the attention of the assisted person towards the light, which provides the light of modulated intensity. Specifically, in case that a regular luminaire providing illumination of the room shall be used for the present invention, this helps to make sure that the concerned person focuses on the luminaire used for providing assistance and calming down. If the color of the emitted light suddenly changes, for example, when starting the assistance routine, a person in the room tends to look into the direction of the changed color. Choosing a color, for example green, that helps to calm down a person even improves the effect of the present invention.

The change of the color information might be independent from measured values of physiological parameters but take into consideration the elapsed time from starting the output of the control signal for causing output of the modulated light in accordance with the above given explanations. According to another preferred embodiment, the control device is configured to generate the control signal including information on a maximum light intensity and/or a minimum light intensity. Alternatively, it is also possible to include information on a relative change of the light intensity into the control signal. The latter is advantageous when starting with an already operating light source from its current light intensity. For example, a room in a hospital where a patient lies in bed, is illuminated by a luminaire mounted on the ceiling. When a calming demand is recognized by the control device, the control device starts generating the control signal and includes information on a relative intensity variation for modulating the light intensity of the luminaire. In contrast, providing information on a maximum light intensity and a minimum light intensity provides the advantage that even in case that the light is operated at a relatively low dimming level, the emission of the modulated light ensures catching the attention of the concerned person. In order to achieve this effect, the maximum light intensity may be chosen to be at least close to the maximum possible light emission by the respective light source. The maximum light intensity and the minimum light intensity may even be chosen in consideration of environmental conditions, for example brightness of ambient light.

Embodiments of the present invention will now be explained with reference to the attached drawings, in which
- Figure 1: is a block diagram of components of an embodiment of a system according to the present invention, including the control device,
- Figure 2: is an exemplary course of an intensity of light emitted by light source in accordance with the respective control signal from the control device using a fixed frequency decrease rate,
- Figure 3: is a second exemplary cause of an intensity of light emitted by a light source in accordance with the respective control signal from the control device using an adapted frequency decrease rate,
- Figure 4: is a simplified flowchart illustrating the method for outputting intensity modulated light for calming down the concerned person.

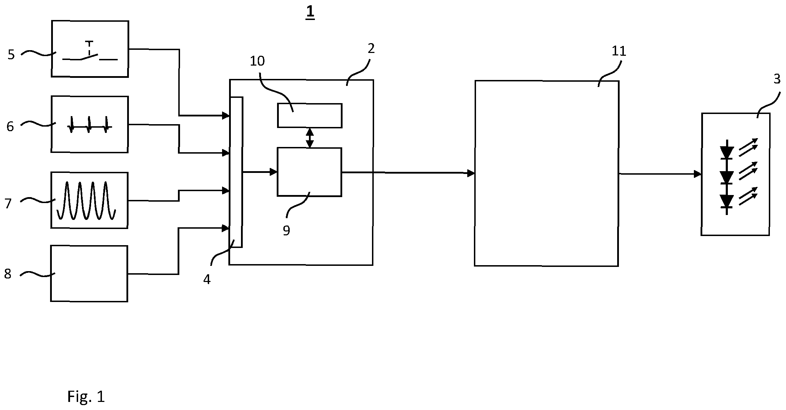

Figure 1 gives an overview over the system according to the present invention. The system 1 comprises a control device 2 for generating and outputting a control signal. The control signal is the basis for controlling light emission of a luminaire 3. The luminaire 3 is a luminaire which is able to emit light at different light intensities and, preferably, with different colors. Preferably, the luminaire 3 comprises a plurality of LEDs constituting the light source, which is driven based on the control signal generated by the control device 2

The control device 2 comprises an interface 4 for receiving input signals from external signal sources. The signal sources can be of different types, depending on the desired functionality of the overall system 1. The system 1 requires only a single signal source in a basic configuration. In the illustrated embodiment, four different signal sources are shown. One possible signal source is a switch comprising a push button 5, which is connected with the interface 4 of the control device 2. When the button 5 is pressed, the interface 4 will receive a respective signal from the button 5. In case an input signal from the button 5 is received by the control device 2, receiving such (trigger) signal will be interpreted as a calming demand by the control device 2. Accordingly, the signal provided by button 5 is indicative of a calming demand, because only in case that a calming assistance is requested by a person the person will operate the button to provide the respective signal to the control device 2

Preferably, the interface 4 is connected with one or more sensors as individual signal sources. One such sensor may be a heart rate sensor 6 communicating a measured heart rate over the interface 4 to the control device 2. Additionally or alternatively, a respiratory rate sensor 7 may be connected to the interface 4. The respiratory rate sensor 7 measures the breathing frequency of a person and communicates the measured frequency to the interface 4. Additionally or alternatively, the sensors may be used for measuring physiological parameters of a person and directly provide respective values to the interface 4 for further processing in the control device 2. These additional or alternative sensors are indicated by the blank sensor 8. For example, sweat on a skin surveys of a person could be identified by such additional or alternative sensor 8.

In the illustrated embodiment, the output from the sensors 6, 7, 8, ... is provided to the control device 2. It is assumed that further processing of the information received in the supplied input signals is performed in the control device 2. However, the further evaluation of the signals received from the physiological sensors 6, 7, 8,... may also be performed outside the control device 2 and the result thereof is then provided to the control device 2. This is especially advantageous, if at least partially an evaluation of the measured values of the physiological census 6, 7, 8,... is performed anyway, in order to monitor the current condition of the concerned person. For the following explanations it is nevertheless assumed that such processing of the information obtained via the interface 4 is performed inside the control device 2 based on signals received from the physiological sensors 6, 7, 8,... providing signals indicative of a calming demand. The signals are indicative of a calming demand, because the measured physiological parameters correlate with stress level of the person.

In the control device 2, the information received via the interface 4 is processed in a processing unit 9. The processing unit 9 generates, based on the information received over the interface 4 a control signal. The control signal generated by the processing unit 9 is output by the control device 2. Based on the control signal output by the control device 2, the luminaire 3 is driven in order to emit modulated light.

The generation of the control signal by the processing unit 9 may be based on information stored in a memory 10, which is connected to the processing unit 9. For example, memory 10 may hold algorithms and/or thresholds in order to determine whether the received signals from the signal sources indicate a stress level in response to which generation of a control signal causing light emission for assisting calming down a person shall be generated. The signals from the signal sources are, in different ways, indicative of the stress level of a concerned person. While this is evident for sensors like the heart rate sensor 6 and the respiratory rate sensor 7, this is also true for the button 5. While the button 5 may be operated by any person including the concerned person, receiving such a signal from the patent 5 is interpreted by the control device 2 is a calming demand, which, in turn means that the pressing of the button numeral 5 is indicative of a stress level which shall initiate assistance for calming the person down.

In the illustrated embodiment, the control signal produced by the control device 2 is supplied to a control gear 11 of the luminaire 3. The control signal may for example may include a dimming level directly providing the control gear 11 with a target dim level value for the output light intensity of the luminaire. The control signal may directly control the control gear 11 by providing target values varying over time in accordance with the desired modulation of light intensity by the luminaire 3. In that case, the information on a frequency decrease is included in the control signal directed by adapting the variation of the target dim level over time.

Alternatively, the control signal may include information on the modulation frequency and its decrease over time, for example by including information on a frequency decrease rate.

The control signal may further include information on the color of the light to be emitted by the luminaire 3. In particular, color information may change over time. According to one embodiment, the color information could set an initial color when starting to generate the control signal and, after a predefined time interval from starting generation of the control signal elapsed, the color information changes, causing another light color to be emitted by the luminaire 3. Alternatively, the color information included in the control signal may change depending on the progress in calming down the person. This is specifically advantageous in case that the actual status of the person can be monitored based on an input received from the physiological sensors like the heart rate sensor 6 and the respiratory rate sensor 7.

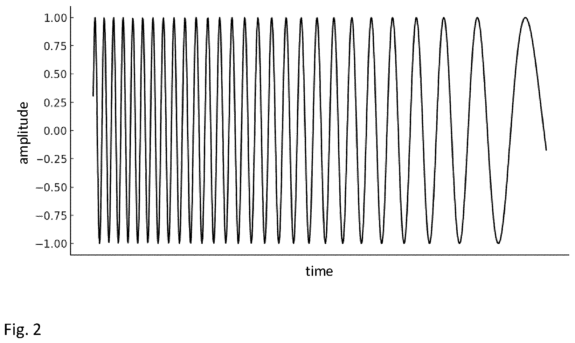

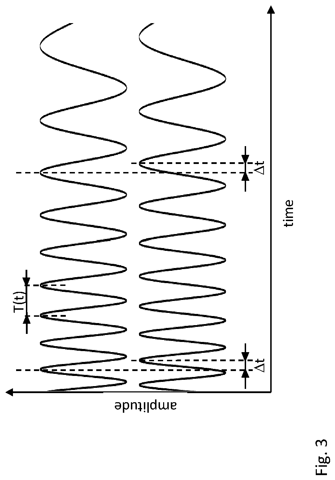

Similarly, it is possible to adjust the actual frequency of the modulation of light intensity to the actual stress level of the concerned person. An actual stress level may be determined by repeatedly determining such stress level from the input signals. Examples for a specific frequency decrease will be explained below with reference to figures 2 and 3.

In case that the control signal generated by the processing unit 9 directly provides the control gear 11 with a dim level over time, the control signal automatically includes information on the maximum intensity level and the minimum intensity level of the modulated light intensity. Accordingly, a maximum dim level and a minimum dim level for the intensity modulation are defined in the control signal.

However, it is also possible that the control signal only includes information on a relative intensity change (relative dim level change). In that case, the control gear 11 will start from its current intensity level and then follow the relative change according to the information included in the control signal received from the control device 2.

The control gear 11 may be any control gear known in the art, which is able to control a luminaire to output light at the desired light intensity (depending on the respective dim level defining the desired light intensity level) and, preferably, with a target light color. In that case, it is possible that in addition to the control signal being input from the control device 2 into the control gear 11, the control gear 11 may receive additional switching, dimming and color information from other lighting controls to define the operational parameter for its regular operation, which means apart from the calming operation described herein. This is particularly useful for situations in which a conventional luminaire is arranged for example on a ceiling of a room in a hospital and this luminaire shall be used in order to assist a patient and calming down.

However, it is also possible to provide a dedicated control gear including the functionality of the control device 2 and the control gear 11 as illustrated in figure 1.

Finally it would also be possible to use the control signal provided by the control device 2 in order to drive a separate dimming unit, for example performing pulse width modulation dimming, between the control gear 11 and the luminaire 3. Assumed that the luminaire 3 is already switched on and power is provided by the control gear 11, such separate dimming unit inserted between the control gear 11 and the luminaire 3 can directly operate on the output drive signal for the luminaire 3 provided by the control gear 11 at least for adjusting the output light intensity of the luminaire 3 by modulating the drive signal from the control gear 11 and reducing the modulation frequency over time.

Figure 2 shows an exemplary course of a control signal including only information on the frequency decrease over time, information on a maximum intensity level and information on a minimum intensity level. It is to be noted, that the units for the amplitude (intensity level) are arbitrary units and only for illustration purposes. For example, 1.00 might equal 100% dim level and minus 1.00 might equal 50% dim level. Further, 0.00 could correspond to a current dim level of the luminaire 3, when it is operated for conventional illumination of a room in a regular operation mode before a calming demand is identified. 1.00 could then again correspond to 100% dim level thereby defining the variation of the intensity level relative to the current intensity of the already operated luminaire. The same amount of variation is then used in order to reduce the dim level to reach the minimum value. Obviously, the latter example is only operable in case that the current dimming level of the regular operation of the luminaire 3 is below 100%.

The diagram shown in figure 2 shows a continuous decrease of the modulation frequency over time. The frequency decrease rate applied here may be stored as a default value in the memory 10. This default frequency rate decrease of the modulation frequency is applied to a starting frequency when the control signal is generated upon recognition of a calming demand. Advantageously, the starting frequency is determined from values received from the physiological sensors 6, 7, ...

In addition to immediately starting the decrease of the modulation frequency when the control signal is generated in response to recognizing the calming demand, it could also be considered to maintain a constant modulation frequency during an initial period of time in the beginning before the decrease of the modulation frequency starts. This might improve the tendency of the concerned person to follow the frequency decrease.

In case that the starting frequency for the modulation of the light intensity is based on physiological parameters, it is preferred to use the respiratory rate measured by the respiratory rate sensor 7 as a starting frequency. It is to be noted that throughout this description, it is always referred to the respiratory rate as an example. However, other physiological aspects may be considered. For example, the heart rate could be referred to. However, the heart rate is faster than the respiratory rate and it might be considered to skip, for example, every second cycle of the heartbeat. This avoids that fast modulation of the light intensity may even have a detrimental effect, because it is perceived to be stroboscopic by the concerned person, especially considering the increased heartbeat rate in situations of a high stress level. For assisting the person in calming down is rather preferred to use low frequencies of modulating the light intensity.

Figure 3 shows a further advantageous embodiment, in which the respiratory rate measured by the respiratory rate sensor 7 is continuously monitored and the modulation frequency and its decrease is associated with the actually measured respiratory rate. In figure 3, the respiratory rate (breathing frequency) is shown in the upper part of the diagram. The lower part of the diagram shows information about the decrease of the frequency of the modulation of light intensity included in the control signal, wherein in the illustrated embodiment the dimming level as a function of time is directly included in the control signal. It can be recognized, that the amplitude of the dimming level follows the amplitude of the values measured by the respiratory rate sensor 7 with a time offset. The maximum of the amplitude of the control signal follows with an offset the sensor signal from the respiratory rate sensor 7. This will cause the person automatically to try to slow down breathing and follow the output light intensity, which is always slightly delayed compared to breathing. The attempt to follow the light intensity change of the luminaire 3 by breathing and synchronism with the modulated light intensity will therefore cause an increase of the time period T(t). In the illustrated embodiment, a constant offset Δt is used. However, this is only provided as one example, and a change of the offset time, for example depending on the actually measured respiratory rate or any other physiological parameter may also be applied.

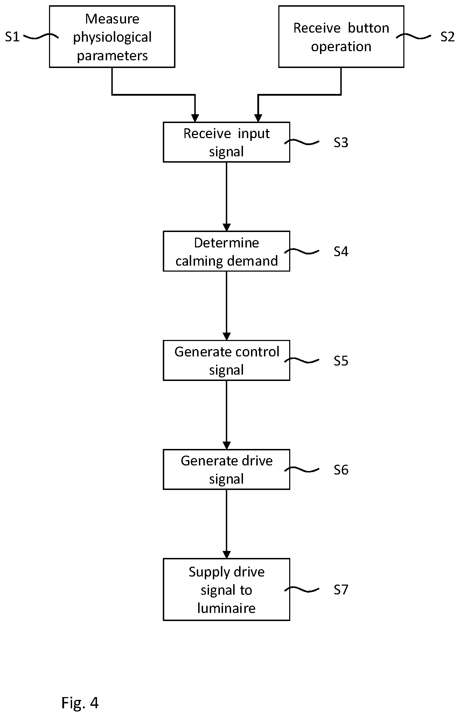

Figure 4 shows a simplified flowchart of the method according to the present invention. It is to be noted that the individual method steps have also been explained above with reference to the respective components of the inventive system 1. In order to avoid unnecessary repetition, it is referred to these details when explaining the major method steps below.

First, the method according to the invention assisting the person in calming down starts with initiating the generation of the control signal. The generation of the control signal, which is then used in order to cause light emission and control the intensity variation of the light emission of the luminaire 3 is started, and the respective calming demand is identified.

There are two possible ways in order to provide a signal indicative of a calming demand to the control device 2: firstly, physiological parameters may be measured by using respective sensors 6, 7, 8.... in step S1. The measured values of the physiological parameters are provided to the control device 2, which receives the input signals in step S3. As mentioned above, directly providing the measured values of the physiological parameters is only one example. Alternatively, preprocessing of the measured values may be performed. In any case, the measured physiological parameters allow to conclude on a stress level of the concerned person and the respectively input signal is therefore indicative of a stress level and, accordingly, a calming demand. Secondly, the concerned person itself, or another person recognizing that the current condition of the concerned person is an increased stress level, may operate a dedicated signal source, for example the button 5. In response to such operation in step S2, the respective signal source supplies the signal, which is indicative of a calming demand again, to the control device 2.

Accordingly, in step S3, the control device 2 receives an input signal indicative of a calming demand. The control device 2 determines in step S4, based on the input signal indicative of the calming demand, that a demand for calming assistance is required. In response to such recognition that the calming a calming demand is required, it starts generating a respective control signal causing light emission assisting the concerned person in calming down. The determination of the calming demand is performed in step S4, and the generation of the control signal starts in step S5.

As explained above in greater detail, generation of the control signal may be performed in different ways. The frequency decrease over time may either follow a predefined course or, alternatively, the decrease rate may be adapted to continuous or repeated measurement of the physiological parameters of the concerned person. The decrease of the modulation frequency of the light intensity therefore either follows a fixed scheme or the actual modulation frequency is repeatedly determined based on the input signals received from the physiological sensors 6, 7, 8,....

The control device 2 outputs the control signal, and in step S6 the control gear 11 generates a drive signal for supplying electric power in line with the information included in the control signal to the luminaire 3. The drive signal is finally supplied to the luminaire 3 in step S7. In response to receiving the respective drive signal the luminaire 3 emits light with modulated intensity according to the modulation frequency and modulation frequency decrease included in the control signal.

Although not specifically repeated, it is evident, that the control signal generation S5 may also comprise including color information into the generated control signal.

## Claims

1. Control device for controlling light emission of a light source (3) to calm down a person, the control device (2) comprising an interface (4) for receiving at least one input signal indicative of a calming demand, wherein the control device (2) is configured to generate a control signal for controlling an amplitude modulation of emitted light when a calming demand is recognized, wherein the control signal includes information on a frequency decrease of the amplitude modulation, and to output the generated control signal.

2. Control device according to claim 1, wherein the control device (2) is configured to determine a stress level by evaluating physiological parameters based on the at least one input signal, and to identify a calming demand from the determined stress level.

3. Control device according to claim 1 or 2, wherein the control device (2) comprises a memory (10) storing a preset frequency decrease rate.

4. Control device according to any one of claims 1 to 2, wherein the control device (2) is configured to repetitively determine the frequency decrease of the amplitude modulation based on the at least one input signal.

5. Control device according to any of the preceding claims, wherein the control device (2) is configured to include color information for controlling the light emission of the light source (3) into the output control signal.

6. Control device according to claim 5, wherein the color information depends at least on one of: a time elapsed from the recognition of a calming demand, on the determined stress level, actual value of a body parameter.

7. Control device according to any of the preceding claims, wherein the control device (2) is configured to generate the control signal including information on a maximum light intensity and/or a minimum light intensity.

8. System for controlling light emission of a light source (3) to calm down a person, the system (1) comprising at least one signal source (5, 6, 7, 8) configured to transmit a signal indicative of a calming demand, a control device (2) according to any one of claims 1 to 7, a light source (3), and a driver (11) for driving the light source (3) according to the control signal output by the control device (2).

9. Method for driving a light source (3) to calm down a person, comprising to following method steps:
- receiving (S3) by a control device (2) at least one input signal indicative of a calming demand,
- analyzing the received at least one signal to recognize a calming demand (S4),
- generating, in case that a calming demand can be recognized by the evaluation of the at least one input signal, a control signal (S5) for controlling an amplitude modulation of emitted light, wherein the control signal includes information on a frequency of the amplitude modulation, and to output the generated control signal,
- outputting the control signal and driving the light source (3) based on the output control signal (S7).

10. Method according to claim 9, wherein the control device (2) determines a stress level by evaluating physiological parameters based on the at least one input signal, and to identify a calming demand (S4) from the determined stress level.

11. Method according to claim 9 or 10, wherein the frequency is decreased at a preset frequency decrease rate.

12. Method according to any one of claims 9 or 10, wherein the control device (2) repetitively determines the frequency decrease of the amplitude modulation based on the at least one input signal (S5).

13. Method according to any of claims 9 to 12, wherein the control device (2) includes color information for controlling the light emission of the light source into the output control signal (S5).

14. Method according to claim 13, wherein the color information depends at least on one of: a time elapsed from the recognition of a calming demand, on the determined stress level, actual value of a body parameter.

15. Method according to any one of claims 9 to 14, wherein the control device (2) generates the control signal (S5) including information on a maximum light intensity and/or a minimum light intensity.
